# EUROPÄISCHE PATENTSCHRIFT

(11) **EP 3 171 827 B1**
(45) Veröffentlichungstag und Bekanntmachung des Hinweises auf die Patenterteilung: **06.06.2018**
(21) Anmeldenummer: 15741166.1
(22) Anmeldetag: 21.07.2015
(51) Int. Cl.: A61F 2/78, A61F 2/80

(54) **PERFORIERTER LINER**
PERFORATED LINER
MANCHON PERFORÉ

(30) Priorität: 23.07.2014 DE 102014011034
(43) Veröffentlichungstag der Anmeldung: 31.05.2017
(73) Patentinhaber: Uniprox GmbH & Co. KG, 07937 Zeulenroda-Triebes (DE)
(72) Erfinder: WALTER, Dennis, 37115 Duderstadt (DE)
(74) Vertreter: Schwahn, Hartmut
(86) Internationale Anmeldenummer: PCT/EP2015/066602
(87) Internationale Veröffentlichungsnummer: WO 2016/012423

(56) Entgegenhaltungen:
- WO-A1-2014/205403
- US-A1- 2007 225 824
- US-A1- 2012 191 218
- US-A1- 2013 035 770
- US-B1- 6 544 292

## Beschreibung

Die Erfindung betrifft einen verbesserten Liner für Prothesen zum Anlegen an einen Extremitätenstumpf. Die Erfindung stellt eine Verbesserung beim Tragen der Prothese dar.

Eine fehlende Gliedmaße des Körpers kann durch eine Prothese ersetzt werden. Die Prothese bildet dabei die Funktion und/oder Gestalt des fehlenden Körperglieds nach. Zur mechanischen Verbindung zwischen Prothese und dem Stumpf der Extremität, zum Beispiel Unterschenkel- oder Oberschenkelstumpf nach Amputation oder Exartikulation, weist die Prothese einen sogenannten Prothesenschaft auf, der den Extremitätenstumpf aufnimmt und die Prothese weitgehend form- und kraftschlüssig an der Extremität fixiert. Gegebenenfalls können zusätzlich Bänder und ähnliche Mittel ein Abrutschen des Prothesenschafts von dem Extremitätenstumpf verhindern.

Zur Fixierung des Prothesenschafts an den Extremitätenstumpf ist dabei besonders ein strumpfförmiger sogenannter Liner vorgesehen. Dieser besteht bekanntermaßen aus einem elastischen Material, Silikonkautschuk oder Polyurethan- oder Chloropren-basiertem Polymer, besonders RTV-Silikon, einer Shore A Härte von 20 bis etwa 50. Der Prothesenliner wird vor dem Anlegen der Prothese über den Extremitätenstumpf gezogen und bildet eine haftende, dichtende und puffernde Zwischenlage zwischen Körper und Prothesenschaft, welche Passungenauigkeiten, die im Laufe des Tragens auftreten können, ausgleicht und gleichzeitig den Tragekomfort erhöht.

In einer Ausgestaltung eines Prothesenschafts ist dieser so an den Extremitätenstumpf spezifisch angeformt, dass eine Fixierung unterstützt wird. Ein Kraftschluss wird erreicht, indem durch haftvermittelnde Lagen zwischen Prothesenschaft einerseits und Hautoberfläche des Extremitätenstumpfs andererseits die Haftreibung erhöht wird. Dabei macht man sich zunutze, dass beim Aufsetzen des Prothesenschafts auf den Extremitätenstumpf Luft ausgepresst wird, die durch die weitgehend dichtende Wirkung der Oberflächen zwischen Extremitätenstumpf und Prothesenschaft nicht mehr ohne weiteres zurückströmen kann, sodass eine Haftung erzielbar ist.

In einem alternativen aber damit grundsätzlich verwandten Prinzip macht man sich primär die Oberflächeneigenschaft des Elastomermaterials des Liners zunutze, welche eine hohe Friktion an einer, insbesondere unbehaarten, Hautoberfläche einer Extremität bedingt. Bekanntermaßen weisen bestimmte Elastomere wie Silikonkautschuk oder Polyurethan- oder Chloropren-basierte Polymere eine besonders innige Haftung an der Haut auf, besonders wenn das Elastomer, insbesondere aufgrund seiner Eigenelastizität, an die Hautoberfläche angedrückt ist. Ein bekannter Prothesenliner haftet - unter Vorspannung über den nackten Extremitätenstumpf - ohne Weiteres an dem Stumpf und kann dort große Scherkräfte aufnehmen. Durch zusätzliche Maßnahmen und Mittel ist der haftende Liner mit dem starren Schaft der Prothese mechanisch verbunden, beispielsweis durch einen distalen Metallsockel an dem Liner, der mit einer entsprechenden Struktur an dem Prothesenschaft kraft- und formschlüssig in Eingriff kommt. Die Prothese wird so auch ohne ein primär sich zwischen Stump und Schaft bildendes Vakuum an diesem fest gehalten.

Um die Haftung zu bewirken, sind Prothesenliner aus mindestens einer luftundurchlässigen Vollmaterialschicht gebildet, welche Extremitätenstumpf und die Innenseite des Prothesenschafts von der Umwelt abdichtet, um ein Vakuum zu bilden. Nachteilig bei bekannten Prothesenlinern aus Elastomermaterial ist generell die Unverträglichkeit von dichtenden Elastomer oder Gummi und der Oberfläche des Hautorgans. Beim Tragen kommt es durch die zwangsläufige Aktivität von Talg- und insbesondere Schweißdrüsen in der lebenden Haut zur nachteiligen Bildung von Flüssigkeit zwischen Haut des Extremitätenstumpfs und Innenseite des Prothesenliners. Dies bewirkt eine subjektive Missempfindung beim Tragen und verringert damit die Compliance für das zu medizinischen Zwecken und aus Gründen der Sicherheit notwendigen Tragen der Prothese. Zum anderen kommt es durch die Flüssigkeitsbildung nachteiligerweise zu einer Verringerung der Haftreibung zwischen Hautoberfläche und Liner, wodurch das Risiko besteht, dass sich beim Tragen der Prothesenschaft von dem Extremitätenstumpf unvermittelt löst oder lockert, was mit gesundheitlichen Gefahren für den Prothesenträger verbunden ist, vor allem durch Verlust der Steuerbarkeit der Prothese, des gegebenenfalls künstlichen Kniegelenks an der Prothese. Unmittelbar wird beim Gehen das Risiko zu stolpern und zu stürzen deutlich erhöht. Weiter tritt bei fehlender Haftung zwischen Liner und Hautoberfläche eine meist lokale, aber erhebliche Reibbelastung der Hautoberfläche auf. Ist die Haut dabei schon von der gebildeten Flüssigkeit aufgeweicht, kommt es zu Hautreizungen und Verletzungen. Grundsätzlich ist die Gegenwart von Flüssigkeit an der Hautoberfläche, auch bei Ruhe problematisch, weil die Hautoberfläche in Mitleidenschat gezogen wird. Dies ist vor allem bei Narben oder Wunden höchst kritisch. Neben allgemeinen Reizungen und Wundheilungsstörungen, können sich auch Infektionen, bakterieller oder pilzlicher Art entwickeln. Prothesenliner gemäß dem Oberbegriff des Anspruchs 1 sind aus dem Stand der Technik bekannt, wie zum Beispiel aus den US 2012/191218 A1, US 2013/035770 A1 und US 2007/225824 A1, die Prothesenliner mit Perforationen für Gas- und Flüssigkeitsaustausch beschreiben. Aufgabe der Erfindung war es, bekannte Prothesenliner derart zu verbessern, dass zum einen die Funktion des Prothesenliners erhalten bleibt, zum anderen aber die von bekannten Prothesenlinern bekannten Nachteile, besonders die Bildung von Flüssigkeit zwischen der Haut des Extremitätenstumpfs und dem angelegten Prothesenliner, vermindert oder ganz verhindert werden.

Dazu stellt die Erfindung einen neuartigen Prothesenliner zum Anlegen an einen Extremitätenstumpf gemäß Anspruch 1 bereit. Dieser weist mindestens eine elastische Linerlage aus Elastomer auf, die zumindest im Bereich der distalen Spitze des Extremitätenstumpfs Poren aufweist. Erfindungsgemäß ist dabei vorgesehen, dass diese Poren im entspannten Zustand der Linerlage im Wesentlichen geschlossen sind und sich durch Dehnung der Linerlage öffnen lassen. Diese Dehnung wird besonders erreicht, wenn der Prothesenliner an dem Stumpf angelegt ist, die Prothese aufgesetzt ist und der Prothesenträger die Prothese in üblicher Weise benutzt, also Quer- und Scherkräfte zwischen Extremität und Prothesenschaft auftreten. Beispielsweise bei einer Unterschenkel- oder Oberschenkelprothese kommt es beim Gehen zu einem periodischen Dehnen und Entdehnen der Linerlage, wodurch sich die erfindungsgemäßen Poren entsprechend mehr oder weniger weit öffnen beziehungsweise schließen.

Der Prothesenliner der Erfindung weist Poren auf, welche zumindest zeitweise eine gasdurchlässige und flüssigkeitsdurchlässige Verbindung zwischen der dem Stumpf zugewandten Innenseite und der Außenseite des Liners herstellen können.

Es zeigt sich, dass durch die erfindungsgemäßen Poren sich nachteiligerweise sonst zwischen Haut des Extremitätenstumpfs und Innenseite des Prothesenliners bildende Flüssigkeit, das heißt besonders Schwitzwasser, durch die Linerlage hindurch nach außen, das heißt von der Hautoberfläche weg, drainieren lässt. Vorteilhafterweise findet diese Drainage besonders bei Bewegung, das heißt bei Dehnung des Prothesenliners statt. Ohne an die Theorie gebunden sein zu wollen findet durch das Zusammenspiel von Eigenelastizität der Linerlage und dehnungsabhänigen Ändern des Querschnitts der erfindungsgemäßen Poren ein Pumpeffekt statt, welcher die gebildete Flüssigkeit vorzugsweise aktiv nach außen bewegt. Vorteilhafterweise wird dadurch eine übermäßige Ansammlung von natürlicherweise gebildeter Flüssigkeit, das heißt besonders Schwitzwasser, an der Hautoberfläche des Extremitätenstumpfs vermieden. Dies empfindet der Prothesenträger unmittelbar als bedeutende Erhöhung des Tragekomforts. Gleichzeitig wird dadurch die Betriebssicherheit und die Nutzungsdauer der Prothese signifikant erhöht.

Überraschenderweise führen die erfindungsgemäßen Poren wider Erwarten nicht zu einer Verminderung der ansonsten angestrebten Vakuumhaftung des Prothesenschafts an dem Extremitätenstumpf. Im Gegenteil, wider Erwarten wird die haftvermittelnde Wirkung des Prothesenliners durch die Poren, vor allem aufgrund der vorteilhaften Drainage von Flüssigkeit, verbessert.

In einer bevorzugten Ausgestaltung sind die Poren nur in dem distalen Abschnitt des Prothesenliners, also im Bereich der Spitze des Extremitätenstumpfs, angeordnet. In einer alternativen Ausgestaltung ist die lokale Dichte, das heißt die Anzahl an Poren pro Fläche, in dem distalen Abschnitt des Prothesenliners höher als in den übrigen Abschnitten des Prothesenliners. In einer bevorzugten Variante ist, alternativ oder zusätzlich, ausgeschlossen, dass in einem proximalen Abschnitt des Prothesenliners Poren vorhanden sind.

In einer anderen Variante sind die Poren über die gesamte Lineroberfläche verteilt. In einer weiteren Variante sind die Poren auf bestimmte Zonen, die mit anatomischen Strukturen der darunter liegenden Extremität korrespondieren, beschränkt, d.h. dort ausschließlich vorhanden, oder dort in hoher Dichte vorhanden. Besonders sind diese Zonen an Stellen hoher Schweißdrüßendichte der Haut. Umgekehrt kann in bestimmten Zonen des Liners das Vorkommen von Poren, beispielsweise aus Gründen der Stabilität, ausgeschlossen sein. Dies sind besonders Zonen hoher mechanischer Zugbelastung, beispielsweise im Bereich der Patella, der vorderen Schienbeinkante oder der Kondylen.

Unter einem "distalen Abschnitt" wird derjenige Bereich des Prothesenliners verstanden, welcher, bezogen auf die Gesamtausdehnung des Prothesenliners, in Längsrichtung, sich über 70 %, bevorzugt 66 %, besonders bevorzugt 40 %, der distalen Seite des Prothesenliners erstreckt. Unter einem "proximalen Abschnitt" wird derjenige Bereich des Prothesenliners verstanden, welcher, bezogen auf die Gesamtausdehnung des Prothesenliners, in Längsrichtung, sich über die proximalen 70 %, bevorzugt 50, besonders bevorzugt 30 %, der proximalen Seite des Prothesenliners erstreckt.

Bevorzugt weisen die Poren in der Linerlage eine mittlere Dichte von 2 bis 20, besonders von 4 bis 16 Poren pro cm² Fläche auf. Abschnitte mit "hoher Dichte" weisen hingegen eine Dichte von etwa 8 bis 20 Poren pro cm² auf; Zonen mit geringer Dichte weisen dabei eine Dichte von etwa 2 bis 8 Poren pro cm² auf.

Der erfindungsgemäße Prothesenliner kann dabei aus allen bekannten zur Verwendung bei Prothesenlinern bekannten Elastomermaterialien gebildet sein. Bevorzugt ist die mindestens eine elastische Linerlage aus einem Elastomer gebildet, welches ausgewählt ist aus Silikonelastomer (Silikonkautschuk), Polyurethanelastomer, Chloroprenelastomer. Besonders bevorzugt ist Silikonelastomer, insbesondere RTV Silikon. Die Shorehärte (A) beträgt vorzugsweise von 20 bis 50. Die Dicke der Linerlage beträgt vorzugsweise 2 bis 4 mm, bei sogenannten Gellinern bis 6 mm.

In besonders bevorzugter Ausgestaltung sind die Poren in der Linerlage durch spanlose Verfahren gebildet. Diese sind bevorzugt ausgewählt aus Bearbeitungsschritten wie Schlitzen oder Stechen. Besonders erfolgt dies durch Einsatz von Messern und/oder Nadeln. Besonders in der Ausbildung der Poren durch diese spanlosen Verfahren wird gewährleistet, dass sich die Poren im entspannten, ungedehnten Zustand des Prothesenliners im Wesentlichen in geschlossenem Zustand befinden und sich erst durch Dehnungsbeanspruchung öffnen lassen. Im erfindungsgemäßen Sinne wird dadurch die Ventilwirkung an der Linerlage besonders gut gewährleistet.

In alternativer Ausgestaltung sind die erfindungsgemäßen Poren in der Linerlage durch sogenannte spanende Verfahren gebildet. Diese sind bevorzugt ausgewählt aus den Bearbeitungsschritten Stanzen und Bohren. Es werden spanende Verfahren als materialabtragende Verfahren verstanden. In dieser Ausgestaltung werden die Poren also unter Materialverlust in Form von Löchern in der Linerlage gebildet.

In einer alternativen Ausgestaltung sind in der Linerlage sowohl durch spanende Verfahren gebildete Löcher als auch durch spanlose Verfahren gebildete Schlitze kombiniert vorhanden.

In einer bevorzugten Variante ist vorgesehen, die Poren ausschließlich oder alternativ in hoher Dichte in denjenigen Bereichen der Linerlage des Prothesenliners vorzusehen, der im angelegten Zustand über denjenigen Bereichen des Extremitätenstumpfs positionierbar ist, wo eine hohe Dichte an Schweißdrüsen vorhanden und damit eine vermehrte Flüssigkeitsbildung zu erwarten ist. In einer alternativen oder zusätzlichen Ausgestaltung befinden sich die Poren ausschließlich oder alternativ in hoher Dichte in denjenigen Bereichen der Linerlage, wo im angelegten Zustand und bei üblicher Benutzung sich dem Schwerkraftvektor folgend die gebildete Flüssigkeit sich an der Innenseite des Prothesenliners sammelt. Dies kann beispielsweise bei einer Ober- oder Unterschenkelprothese die distale Spitze des Extremitätenstumpfs sein.

In einer alternativen oder zusätzlichen Ausgestaltung sind die erfindungsgemäßen Poren in Form von Mikroporen in einer Linerlage ausgebildet, die selbst ein offenporiger Elastomerschaum ist oder diesen enthält. In einer Variante davon weist die Linerlage sowohl offenporige Abschnitte als auch geschlossenzellige Abschnitte und/oder zellfreie Abschnitte auf. Fertigungstechnisch kann dies beispielsweise durch kombiniertes Vergießen von schaumfähigen und nichtschaumfähigen Elastomervorläufern in entsprechende Formen erfolgen. Denkbar ist hier auch eine Kombination von porigem geschäumten Elastomer und durch spanende und/oder spanlose Verfahren wie vorstehend beschrieben gebildeten Poren.

In einer besonderen Ausgestaltung ist vorgesehen, dass an der Außenseite der Linerlage zusätzlich eine Lage aus Textilmaterial angeordnet ist. Diese kann speziell ausgebildet sein, den Gasaustausch an den Poren zu ermöglichen oder zu erleichtern. Alternativ oder zusätzlich kann die Textillage speziell ausgebildet sein, aus den Poren austretende Flüssigkeit aufzunehmen und bevorzugt weiterzutransportieren oder zu verteilen. Dadurch wird bevorzugt erreicht, dass die Verwendung zwischen Extremitätenstumpf und Prothesenschaft weiterhin vollständig von der Umgebung abgeschlossen sein kann, was die Haftung des Prothesenschafts weiter verbessert. Dabei ist besonders vorgesehen, dass das Textilmaterial an der Außenseite des Prothesenliners lokal austretende Flüssigkeit an der Außenseite verteilt, aufnimmt und speichert. An sich bekannte Vorgänge und Maßnahmen können nun, beispielsweise in Ruhephasen des Prothesenträgers, insbesondere wenn kein neuer Schweiß nachgebildet wird, zum langsamen Ableiten oder Abtrocknen der gebildeten Flüssigkeit dienen.

In einer bevorzugten Variante davon ist die Textillage separat ausgebildet und von der Linerlage des Prothesenliners körperlich getrennt und von dieser abnehmbar. In einer alternativen Variante ist die Textillage mit der Linerlage des Prothesenliners zu einer Einheit fest verbunden. Dazu kann das Textilmaterial mit der Linerlage verklebt sein oder die Linerlage aus Elastomer ist auf dem Textilmaterial aufpolymerisiert.

Gegenstand der Erfindung ist auch die Verwendung des vorstehend charakterisierten Prothesenliners zum Zwecke der Vermeidung oder Verminderung der Bildung von Schwitzwasser an einem Extremitätenstumpf eines Patienten bei angelegter Prothese. Gegenstand ist auch die Verwendung eines in den vorstehenden Ansprüchen charakterisierten Prothesenliners zur Verlängerung der effektiven Tragedauer der Prothese an einem Patienten.

Die Erfindung wird durch die nachstehenden Ausführungsbeispiele näher beschrieben, ohne dass diese beschränkend zu verstehen sind.

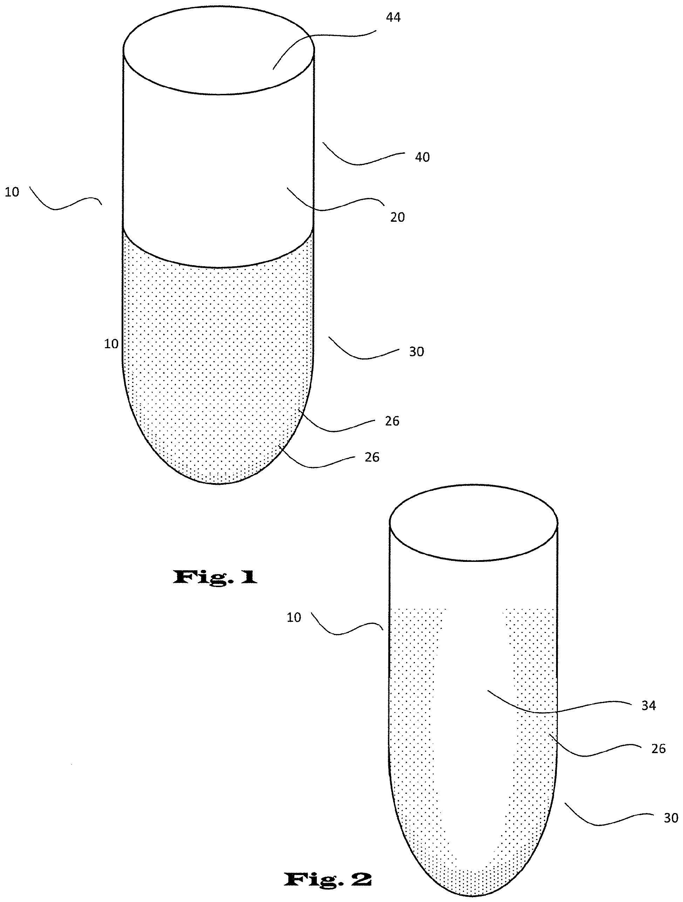

Figur 1 zeigt eine schematische Darstellung des Aufbaus des erfindungsgemäßen Prothesenliners 10. Dieser ist aus zumindest einer elastischen Linerlage 20 aus Elastomermaterial gebildet. Erfindungsgemäß weist die Linerlage zumindest in einem distalen Abschnitt 30 Poren 26 auf. Der Prothesenliner 10 bildet eine strumpfartige Hülse, die an den proximalen Abschnitt 40 eine Öffnung 44 aufweist, welche über den Extremitätenstumpf gezogen wird.
Figur 2 zeigt in Darstellung gemäß Figur 1 einen entsprechenden Prothesenliner 10, dessen Linerlage 20 zumindest in einem distalen Abschnitt 30 Poren 26 aufweißt, nicht aber in bestimmten Zonen 34, welche besonderer mechanischer Belastung ausgesetzt sein können.

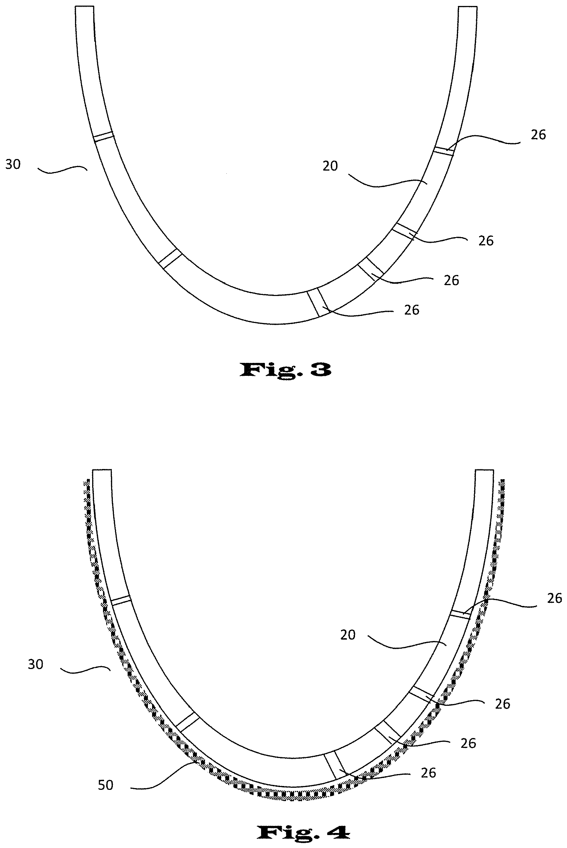

Die Figuren 3 und 4 zeigen eine schematische Detailansicht des Prothesenliners 10 nach Figur 1 oder 2. Zumindest im Bereich des distalen Abschnitts 30 des Prothesenliners sind in der Linerlage 20 Poren 26 gebildet. Figur 3 zeigt eine bevorzugte Ausgestaltung, wobei die Linerlage 20 nach außen hin mit einer zusätzlichen Schicht aus Textilmaterial 50 belegt ist.

## Patentansprüche

1. Prothesenliner (10) zum Anlegen an einen Extremitätenstumpf, mit einer elastischen Linerlage (20) aus Elastomer, wobei die Linerlage (20) zumindest in einem distalen Abschnitt (30) Poren (26) aufweist, **dadurch gekennzeichnet, dass** diese Poren (26) im entspannten Zustand der Linerlage (20) im Wesentlichen geschlossen und durch Dehnung der Linerlage (20) an dem Stumpf bei Bewegung öffenbar sind.

2. Prothesenliner nach Anspruch 1, wobei diese Poren (26) bei Dehnung der Linerlage (20) zumindest zeitweise eine gas- und flüssigkeitsdurchlässige Verbindung zwischen der dem Stumpf zugewandten Innenseite (22) und der Außenseite (24) des Liners herstellen.

3. Prothesenliner nach einem der vorstehenden Ansprüche, wobei an der Außenseite (24) der Linerlage (20) ein Textilmaterial (50) angeordnet ist, das speziell ausgebildet ist, den Gasaustausch an den Poren (26) zu ermöglichen und/oder aus den Poren (26) austretende Flüssigkeit aufzunehmen.

4. Prothesenliner nach Anspruch 3, wobei das Textilmaterial (50) von der Linerlage (20) körperlich getrennt und abnehmbar ist.

5. Prothesenliner nach Anspruch 3, wobei das Textilmaterial (50) mit der Linerlage (20) fest verbunden ist.

6. Prothesenliner nach einer der vorstehenden Ansprüche, wobei die Poren (26) in der Linerlage (20) in einer mittleren Dichte von 2 bis 20 pro cm² angeordnet sind.

7. Prothesenliner nach einem der vorstehenden Ansprüche, wobei die Poren (26) nicht in einem proximalen Abschnitt (40) des Liners vorhanden sind.

8. Prothesenliner nach einem der vorstehenden Ansprüche, wobei das Elastomer ausgewählt ist aus Silikonelastomer und Polyurethanelastomer.

9. Prothesenliner nach einem der vorstehenden Ansprüche, wobei die Poren (26) in der Linerlage (20) durch spanlose Verfahren, ausgewählt aus Schlitzen und Stechen, gebildete Schlitze sind.

10. Prothesenliner nach einem der vorstehenden Ansprüche, wobei die Poren (26) in der Linerlage (20) durch spanende Verfahren, ausgewählt aus Stanzen und Bohren, gebildete Löcher sind.

11. Prothesenliner nach einem der vorstehenden Ansprüche, wobei die Poren (26) in Form von Poren in der Linerlage (20) als offenporiger Elastomerschaum gebildet sind.

12. Verwendung eines Prothesenliners (10) nach einem der Ansprüche 1 bis 11 zur Vermeidung oder Verminderung der Ansammlung von Schwitzwasser an einem Extremitätenstumpf (40) bei daran angelegter Prothese.

13. Verwendung eines Prothesenliners (10) nach einem der Ansprüche 1 bis 11 zur Verlängerung der effektiven Tragedauer einer Prothese.

## Claims

1. Prosthesis liner (10) for application to a limb stump, with an elastic liner layer (20) of elastomer, wherein the liner layer (20) has pores (26) in at least one distal portion (30) thereof, **characterized in that** these pores (26) are substantially closed in the relaxed state of the liner layer (20) and become opened by stretching of the liner layer (20) on the stump during movement.

2. Prosthesis liner according to claim 1, wherein the pores (26) can produce an at least temporary gas-permeable and moisture-permeable connection between the inside (22) of the liner facing the stump and the outside (24) of the liner upon a stretching of the liner layer (20).

3. Prosthesis liner according to one of the proceeding claims, wherein a textile material (50) is arranged on the outside (24) of the liner layer (20), said textile material being specially designed to enable gas exchange at the pores (26) and/or to take up moisture emerging from the pores (26).

4. Prosthesis liner according to claim 3, wherein the textile material (50) is physically separate and can be removed from the liner layer (20).

5. Prosthesis liner according to claim 3, wherein the textile material (50) is firmly connected to the liner layer (20).

6. Prosthesis liner according to one of the preceding claims, wherein the pores (26) are arranged in the liner layer (20) in a mean density of 2 to 20 per cm².

7. Prosthesis liner according to one of the preceding claims, wherein the pores (26) are not present in a proximal portion (40) of the liner.

8. Prosthesis liner according to one of the preceding claims, wherein the elastomer is selected from silicon elastomer and polyurethane elastomer.

9. Prosthesis liner according to one of the preceding claims, wherein the pores (26) are slits formed in the liner layer (20) by chipless processes, selected from slitting and piercing.

10. Prosthesis liner according to one of the preceding claims, wherein the pores (26) are holes formed in the liner layer (20) by chip-removing processes, selected from punching and drilling.

11. Prosthesis liner according to one of the preceding claims, wherein the pores (26) are formed as pores in the liner layer (20) as an open-pore elastomer foam.

12. Use of a prosthesis liner (10) according to one of claims 1 to 11 for avoidance or lessening the accumulation of sweat on a limp stump (40) when a prosthesis is applied.

13. Use of a prosthesis liner (10) according to one of claims 1 to 11 for extending the length of wearing of a prosthesis.

## Revendications

1. Manchon de prothèse (10) destiné à être appliqué sur un moignon de membre, avec une couche de manchon élastique (20) en élastomère, la couche de manchon (20) ayant des pores (26) au moins dans une partie distale (30), **caractérisé en ce que** ces pores (26) sont essentiellement fermées dans l'état détendu de la couche de manchon (20), et peuvent être ouverts dans le cas d'un mouvement en étirant la couche de manchon (20) sur le moignon de membre.

2. Manchon de prothèse selon la revendication 1, dans lequel ces pores (26) au moins temporairement établissent une connexion perméable à gaz et liquides entre le côté intérieur (22) faisant face au moignon de membre et le côté extérieur (24) du manchon lorsque la couche de manchon (20) est étirée.

3. Manchon de prothèse selon l'une quelconque des revendications précédentes, dans lequel un matériau textile (50) est arrangé au côté extérieur (24) de la couche de manchon (20), le matériau étant spécifiquement conçu pour permettre l'échange gazeux sur les pores (26) et/ou pour absorber du liquide s'échappant des pores (26).

4. Manchon de prothèse selon la revendication 3, dans lequel le matériau textile (50) est physiquement séparé et amovible de la couche de manchon (20).

5. Manchon de prothèse selon la revendication 3, dans lequel le matériau textile (50) est fermement attaché à la couche de manchon (20).

6. Manchon de prothèse selon l'une quelconque des revendications précédentes, dans lequel les pores (26) dans la couche de manchon (20) sont arrangés avec une densité moyenne de 2 à 20 par cm².

7. Manchon de prothèse selon l'une quelconque des revendications précédentes, dans lequel les pores (26) ne sont pas prévus dans une partie proximale (40) du manchon.

8. Manchon de prothèse selon l'une quelconque des revendications précédentes, dans lequel l'élastomère est choisi entre élastomère de silicone et élastomère de polyuréthane.

9. Manchon de prothèse selon l'une quelconque des revendications précédentes, dans lequel les pores (26) dans la couche de manchon (20) sont des fentes formées par procédés sans enlèvement de matière choisis parmi fendage et piquage.

10. Manchon de prothèse selon l'une quelconque des revendications précédentes, dans lequel les pores (26) dans la couche de manchon (20) sont des fentes formées par procédés par enlèvement de matière choisis parmi poinçonnage et alésage.

11. Manchon de prothèse selon l'une quelconque des revendications précédentes, dans lequel les pores (26) sont formés sous forme des pores dans la couche de manchon (20) en tant que mousse élastomère à pores ouverts.

12. Utilisation d'un manchon de prothèse (10) selon l'une quelconque des revendications 1 à 11 pour empêcher ou réduire l'accumulation d'eau de condensation sur un moignon de membre (40) lorsque la prothèse est montée sur celui-ci.

13. Utilisation d'un manchon de prothèse (10) selon l'une quelconque des revendications 1 à 11 destinée à prolonger la durée de port effectif d'une prothèse.
